# EUROPÄISCHE PATENTSCHRIFT

(11) **EP 3 468 622 B1**
(45) Veröffentlichungstag und Bekanntmachung des Hinweises auf die Patenterteilung: **30.10.2024**
(21) Anmeldenummer: 17731497.8
(22) Anmeldetag: 08.06.2017
(51) Int. Cl.: A61L 2/08, G01N 21/63, A61L 2/10

(54) **VERFAHREN ZUM BEAUFSCHLAGEN EINER FLÜSSIGKEIT MIT BESCHLEUNIGTEN ELEKTRONEN**
METHOD FOR IMPINGING A LIQUID WITH ACCELERATED ELECTRONS
PROCÉDÉ POUR SOUMETTRE UN LIQUIDE À L'ACTION D'ÉLECTRONS ACCÉLÉRÉS

(30) Priorität: 09.06.2016 DE 102016110672
(43) Veröffentlichungstag der Anmeldung: 17.04.2019
(73) Patentinhaber: Fraunhofer-Gesellschaft zur Förderung der angewandten Forschung e.V., 80686 München (DE)
(72) Erfinder: SCHÖNFELDER, Jessy, 01309 Dresden (DE); RÖGNER, Frank-Holm, 01277 Dresden (DE); PORTILLO, Javier, 01277 Dresden (DE); KUBUSCH, Jörg, 01279 Dresden (DE)
(86) Internationale Anmeldenummer: PCT/EP2017/064034
(87) Internationale Veröffentlichungsnummer: WO 2017/211990

(56) Entgegenhaltungen:
- DE-A1- 102013 109 390
- DE-A1- 102015 117 939
- DE-A1- 102015 117 939

## Beschreibung

Die Erfindung betrifft ein Verfahren zum Beaufschlagen einer Flüssigkeit mit beschleunigten Elektronen, bei dem die Dosis der in die Flüssigkeit eingetragenen Energie überprüft und/oder eingestellt werden kann.

Es sind verschiedene Verfahren bekannt, bei denen Flüssigkeiten mit beschleunigten Elektronen beaufschlagt werden, um schadhafte Mikroorganismen in den Flüssigkeiten zu inaktivieren. In US 4 230 947 A wird eine Flüssigkeit aufgrund des Flüssigkeitsstandes in einem Zuführungsrohrsystem auf ein Plateau gehoben, von dem es als Flüssigkeitsvorhang hinunterfällt. Während des freien Falls wird dieser Vorhang mit beschleunigten Elektronen beaufschlagt. Aus US 3 988 588 A sind Verfahren bekannt, bei denen eine trichterförmige Einrichtung verwendet wird, bei der durch Überlauf oder durch Rotation ein schichtförmiges Volumen einer Flüssigkeit geformt wird, welches dann durch beschleunigte Elektronen beaufschlagt wird. Mit beiden Verfahren ist es jedoch nicht möglich, die in die Flüssigkeit eingetragene Energiedosis zu überwachen bzw. einzustellen.

In DE 10 2013 109 390 A1 ist ein Verfahren beschrieben, bei dem ein Verpackungsmaterial für flüssige oder feste Stoffe sterilisiert werden soll. Hierbei wird am Verpackungsmaterial ein Markermaterialfeld angebracht, welches mindestens einen anorganischen Leuchtstoff umfasst. Das Markermaterialfeld wird mit Elektronen bestrahlt, wodurch die Lumineszenzlebensdauer des Leuchtstoffs verändert wird. Anhand der Veränderung der Lumineszenzlebensdauer des Leuchtstoffs kann festgestellt werden, ob das Markermaterialfeld hinreichend mit Elektronen bestrahlt wurde. Bei diesem Verfahren kann die Energiedosis, die in das Markermaterialfeld eingetragen wurde, exakt ermittelt bzw. eingestellt werden. Eine Aussage darüber, welche Energiedosis tatsächlich in den festen oder flüssigen Stoffen appliziert wurde, die vom Verpackungsmaterial umschlossen sind, kann hingegen nur näherungsweise getroffen werden. DE102015117939 A1 offenbart ein Verfahren, in welchem Partikel, die Leuchtstoffe umfassen, in einem Gefäß - frei beweglich und statistisch verteilt - suspendiert vorliegen, mit beschleunigten Elektronen beaufschlagt und mit niederenergetischer Strahlung zu Fotolumineszenz angeregt werden.

Der Erfindung liegt daher das technische Problem zugrunde, ein Verfahren zum Beaufschlagen einer Flüssigkeit mit beschleunigten Elektronen zu schaffen, mittels dessen die Nachteile aus dem Stand der Technik überwunden werden können. Insbesondere soll es mit dem erfindungsgemäßen Verfahren auch möglich sein, die Dosis beim Beaufschlagen einer Flüssigkeit beschleunigter Elektronen zu überprüfen und/oder einzustellen.

Die Lösung des technischen Problems ergibt sich durch Gegenstände mit den Merkmalen des Patentanspruchs 1. Weitere vorteilhafte Ausgestaltungen der Erfindung ergeben sich aus den abhängigen Ansprüchen.

Beim erfindungsgemäßen Verfahren wird zunächst eine Flüssigkeit bereitgestellt, die mit beschleunigten Elektronen beaufschlagt werden soll. Mit dieser Flüssigkeit werden Partikel vermischt, welche mindestens einen Leuchtstoff aufweisen, wobei die Partikel derart ausgebildet werden, dass die Partikel nach dem Vermischen dispergiert in der Flüssigkeit vorliegen. Als dispergiert im Sinne der Erfindung liegen Partikel dann innerhalb einer Flüssigkeit vor, wenn deren Sink- bzw. Auftriebsgeschwindigkeit innerhalb eines nicht fließenden Volumens der Flüssigkeit kleiner als 100 nm/s ist. Anschließend wird die mit den Partikeln vermischte Flüssigkeit mit einer elektromagnetischen Strahlung beaufschlagt, welche den mindestens einen Leuchtstoff zum Lumineszieren anregt. Eine solche elektromagnetische Strahlung kann beispielsweise Licht, welches mit einem menschlichen Auge erfassbar ist oder auch UV-Strahlung sein. Während und/oder unmittelbar nach dem Beaufschlagen der Flüssigkeit mit der elektromagnetischen Strahlung wird mittels eines Detektors ein Istwert für mindestens eine, die Lumineszenz des Leuchtstoffs charakterisierende, physikalische Größe erfasst und an eine Auswerteeinrichtung weitergeleitet. Eine die Lumineszenz des Leuchtstoffs charakterisierende physikalische Größe kann beispielsweise die Lumineszenzlebensdauer, die Intensität mindestens einer Wellenlänge der Lumineszenz oder auch die Wellenlänge sein, bei der die Lumineszenz ihr Intensitätsmaximum ausbildet. Des Weiteren wird beim erfindungsgemäßen Verfahren die mit Partikeln vermischte Flüssigkeit und somit auch der Leuchtstoff, der mit der Flüssigkeit vermischten Partikel, mit beschleunigten Elektronen beaufschlagt. Durch das Beaufschlagen eines Leuchtstoffs mit beschleunigten Elektronen werden Parameter physikalischer Größen, die die Lumineszenz des Leuchtstoffs charakterisieren, verändert. Erfindungsgemäß wird daher die mit Partikeln vermischte Flüssigkeit so lange mit beschleunigten Elektronen beaufschlagt, bis der mittels des Detektors kontinuierlich oder in Zeitabständen erfasste Istwert für die mindestens eine die Fluoreszenz des Leuchtstoffs charakterisierende Größe einem Sollwert entspricht. Auf diese Weise kann die Dosis, mit welcher eine Flüssigkeit mit beschleunigten Elektronen beaufschlagt werden soll, überwacht und eingestellt werden.

Die vorliegende Erfindung wird nachfolgend anhand von Ausführungsbeispielen näher erläutert. Die Fig. zeigen:
- Fig. 1: eine schematische Darstellung einer Vorrichtung zum Ausführen des erfindungsgemäßen Verfahrens;
- Fig. 2: eine schematische Darstellung einer alternativen Vorrichtung zum Ausführen des erfindungsgemäßen Verfahrens.

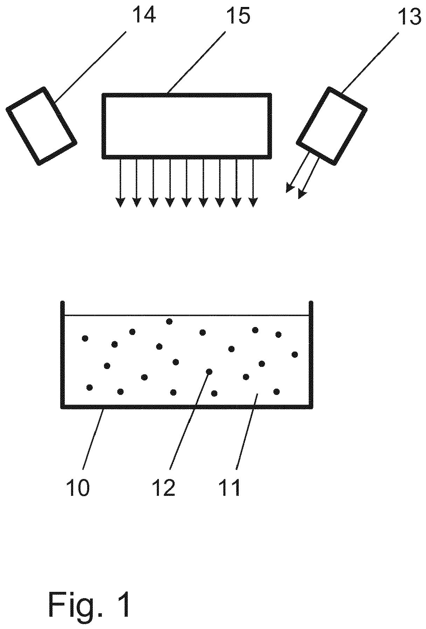

Eine in Fig. 1 schematisch dargestellte Vorrichtung zum Ausführen des erfindungsgemäßen Verfahrens umfasst ein Gefäß 10, in welchem eine beispielhaft als Impfstoff ausgebildete Flüssigkeit 11 bereitgestellt wird. Das Gefäß 10 besteht aus einem nicht flexiblen Material. Alternativ kann sich die Flüssigkeit 11 beim Durchführen des erfindungsgemäßen Verfahrens auch in einem Behältnis aus einem flexiblen Material befinden. So kann ein solches Behältnis beispielsweise auch als Folienbeutel ausgebildet sein.

Die als Impfstoff ausgebildete Flüssigkeit 11 ist mit Mikroorganismen wie zum Beispiel Viren durchsetzt, welche durch das Beaufschlagen mit beschleunigten Elektronen inaktiviert werden sollen. Für das Inaktivieren des Impfstoffs ist eine bestimmte Dosis erforderlich, die nicht zu gering sein darf, um möglichst alle Mikroorganismen im Impfstoff zu inaktivieren. Als Dosis ist hierbei die Energiemenge anzusehen, die beim Beaufschlagen mit beschleunigten Elektronen pro Masseeinheit der Flüssigkeit 11 absorbiert wird. Die Dosis darf aber auch nicht zu hoch sein, weil dadurch die Wirksamkeit des Impfstoffs negativ beeinflusst werden könnte. Welche Dosis bei einem jeweiligen Anwendungsfall optimal ist, lässt sich in Laborversuchen ermitteln.

Erfindungsgemäß wird die Flüssigkeit vor dem Beaufschlagen mit beschleunigten Elektronen mit Partikeln 12 vermischt, die mindestens einen Leuchtstoff enthalten. Dabei können die für das erfindungsgemäße Verfahren verwendeten Partikel 12 vollständig aus dem Leuchtstoff bestehen oder alternativ aus einem Basismaterial, an dessen Oberfläche der Leuchtstoff aufgetragen und/oder in das der Leuchtstoff eingebettet wird. Bei einer Ausführungsform bestehen die Partikel aus einem biokompatiblen Material. Dies ist besonders vorteilhaft, wenn das erfindungsgemäße Verfahren an Impfstoffen, wie im Ausführungsbeispiel beschrieben, durchgeführt wird. Bei einer weiteren Ausführungsform umschließt das Basismaterial vollständig den mindestens einen Leuchtstoff.

Für das erfindungsgemäße Verfahren sind als Leuchtstoff alle aus dem Stand der Technik bekannten Leuchtstoffe geeignet, die sich durch das Beaufschlagen mit einer elektromagnetischen Strahlung zum Lumineszieren anregen lassen. Der mindestens eine Leuchtstoff kann beispielsweise sein: ein Cyanat, Rhodamin oder ein Derivat davon, ein Oxid, ein Oxihalogenid, ein Sulfid, ein Oxisulfid, ein Sulfat, ein Oxisulfat, ein Selenid, ein Nitrid, ein Oxinitrid, ein Nitrat, ein Oxinitrat, ein Phosphid, ein Phosphat, ein Carbonat, ein Silikat, ein Oxisilikat, ein Vanadat, ein Molybdat, ein Wolframat, ein Germanat, ein Oxigermanat oder ein Halogenid der Elemente Li, Na, K, Rb, Mg, Ca, Sr, Sc, Y, La, Ti, Zr, Hf, Nb, Ta, Zn, Gd, Lu, Al, Ga und/oder In. Für das erfindungsgemässe Verfahren beinhalten der Leuchtstoff oder zumindest einer der Leuchtstoffe ein oder mehrere Ionen aus der Gruppe In+, Sn²⁺, Pb²⁺, Sb³⁺, Bi³⁺, Ce³⁺, Ce⁴⁺, Pr³⁺, Nd³⁺, Sm²⁺, Sm³⁺, Eu²⁺, Eu³⁺, Gd³⁺, Tb³⁺, Dy³⁺, Ho³⁺, Er³⁺, Tm²⁺, Tm³⁺, Yb²⁺, Yb³⁺, Ti³⁺, V²⁺, V³⁺, V⁴⁺, Cr³⁺, Mn²⁺, Mn³⁺, Mn⁴⁺, Fe³⁺, Fe⁴⁺, Fe⁵⁺, Co³⁺, Co⁴⁺, Ni²⁺, Cu⁺, Ru²⁺, Ru³⁺, Pd²⁺, Ag⁺, Ir³⁺, Pt²⁺ und Au⁺.

Die Partikel 12 werden für das erfindungsgemäße Verfahren derart ausgebildet, dass diese nach dem Vermischen mit der Flüssigkeit 11 dispergiert in der Flüssigkeit 11 vorliegen. Dies kann im Wesentlichen mittels zweier Parameter eingestellt werden, mit der Größe und/oder der Dichte der Partikel 12. Partikel 12 werden bei einer Ausführungsform des erfindungsgemäßen Verfahrens mit einer Größe im Nanometer- oder Mikrometerbereich ausgebildet. Es ist auch vorteilhaft, wenn die Partikel 12 eine Dichte aufweisen, welche der Dichte der Flüssigkeit 11 entspricht. Weisen die Partikel 12 neben dem Leuchtstoff auch noch ein Basismaterial auf, können beispielsweise Kunststoffe als Basismaterial verwendet werden, weil Kunststoffe mit einer Dichte, ähnlich der Dichte von Flüssigkeiten, hergestellt werden können. Im zu Fig. 1 beschriebenen Ausführungsbeispiel bestehen die Partikel 12 aus einem als Kunststoff ausgebildeten Basismaterial, an dessen Oberfläche bei einer Hälfte der Partikel ein unter der Bezeichnung "FITC" bekannter Leuchtstoff und bei der anderen Hälfte der Partikel ein unter der Bezeichnung "PE" bekannter Leuchtstoff aufgetragen sind.

Die Vorrichtung aus Fig. 1 umfasst ferner eine Einrichtung 13, zum Aussenden einer elektromagnetischen Strahlung, mit welcher die Flüssigkeit 11 und somit auch die darin dispergiert vorliegenden Partikel 12 beaufschlagt werden und welche den in den Partikeln 12 enthaltenen Leuchtstoff zum Lumineszieren anregt. Bei einer Ausführungsform wird der Leuchtstoff vollständig vom Basismaterial der Partikel umschlossen, wobei das Basismaterial transparent gegenüber der elektromagnetischen Strahlung ist. Die elektromagnetische Strahlung wird bei einer Ausführungsform pulsförmig von der Einrichtung 13 ausgesendet.

Während und/oder unmittelbar nach dem Beaufschlagen der Flüssigkeit 11 mit der elektromagnetischen Strahlung wird mittels eines Detektors 14 ein Istwert für die Lumineszenzlebensdauer ermittelt, an eine in Fig. 1 nicht dargestellte Auswerteeinrichtung weitergeleitet und dort mit einem Sollwert für die Lumineszenzlebensdauer verglichen.

Mittels eines Elektronengenerators 15 werden beschleunigte Elektronen erzeugt und mit diesen die Flüssigkeit 11 und die sich darin befindenden Partikel 12 beaufschlagt, wodurch die Lumineszenzlebensdauer des sich in den Partikeln befindenden Leuchtstoffs verändert wird. Als Elektronengenerator 15 kann beim erfindungsgemäßen Verfahren zum Beispiel ein Bandstrahler oder ein Flächenstrahler verwendet werden.

Das Beaufschlagen der Flüssigkeit 11 mit beschleunigten Elektronen erfolgt so lange, bis der mittels des Detektors 14 erfasste Istwert für die Lumineszenzlebensdauer dem Sollwert entspricht, womit dann die zuvor in Laborversuchen ermittelte Dosis, mit der die Flüssigkeit 11 mittels beschleunigter Elektronen beaufschlagt werden soll, erreicht ist.

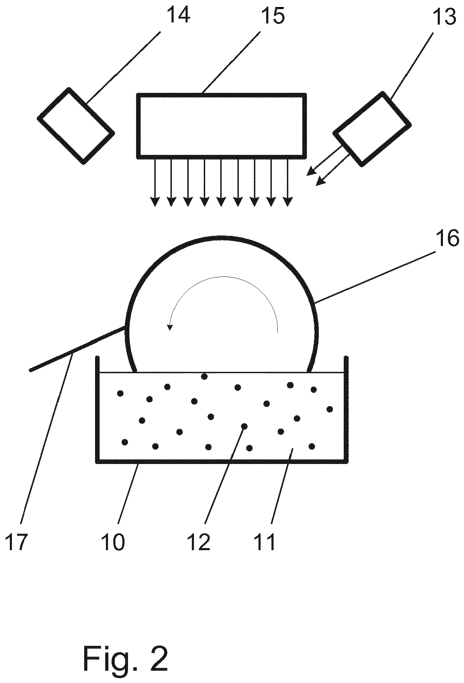

In Fig. 1 ist eine Vorrichtungsanordnung dargestellt, mittels der ein Flüssigkeitsvolumen mit beschleunigten Elektronen beaufschlagt werden kann, welches keine Relativbewegung zum Elektronengenerator 15, zur Einrichtung 13 und zum Detektor 14 aufweist. Eine alternative Vorrichtung ist in Fig. 2 schematisch dargestellt, mittels der ein Flüssigkeitsvolumen mit beschleunigten Elektronen beaufschlagt werden kann, welches eine Relativbewegung zum Elektronengenerator 15, zur Einrichtung 13 und zum Detektor 14 ausführt.

Die in Fig. 2 dargestellte Vorrichtung weist zunächst alle Merkmale der Vorrichtung aus Fig. 1 auf. Zusätzlich umfasst die in Fig. 2 dargestellte Vorrichtung eine Walze 16, welche teilweise in die Flüssigkeit 11 hineinragt und sich entgegengesetzt zum Uhrzeigersinn dreht. Dadurch wird ein Flüssigkeitsfilm an der Oberfläche der Walze 16 ausgebildet, der mit voranschreitender Drehung der Walze 16 durch einen Wirkbereich von Einrichtung 13, Detektor 14 und Elektronengenerator 15 geführt und schließlich von einem Abstreifer 17 wieder von der Walze 16 entfernt wird. Auf diese Weise wird der Flüssigkeitsfilm und die sich darin befindenden Partikel 12 mit der elektromagnetischen Strahlung der Einrichtung 13 beaufschlagt, wodurch der Leuchtstoff der Partikel 12 zum Lumineszieren angeregt wird. Der Detektor 14 ermittelt einen Istwert für die Lumineszenzlebensdauer, welcher in einer Auswerteeinrichtung mit einem Sollwert verglichen wird. Der Flüssigkeitsfilm auf der Walze 16 wird außerdem mit beschleunigten Elektronen des Elektronengenerators 15 beaufschlagt, bis der Istwert der Lumineszenzlebensdauer dem Sollwert entspricht.

Die durch beschleunigte Elektronen applizierte Dosis, mit denen ein Abschnitt des Flüssigkeitsfilms auf der Walze 16 beaufschlagt wird, kann bei einer Vorrichtung gemäß Fig. 2 eingestellt werden, indem beispielsweise die Drehgeschwindigkeit der Walze 16 und/oder die Leistung des Elektronengenerators 15 in Abhängigkeit von einem erfassten Istwert für die Lumineszenzlebensdauer geregelt werden. Wenn der Elektronengenerator 15 gepulst betrieben wird, kann zusätzlich oder alternativ auch die Länge der Pulse und/oder die Länge der Pulspausen in Abhängigkeit von einem erfassten Istwert für die Lumineszenzlebensdauer geregelt werden.

Die in den Fig. 1 und 2 dargestellten Vorrichtungen sind lediglich beispielhaft angeführt. Zum Durchführen des erfindungsgemäßen Verfahrens sind alternativ auch alle anderen Vorrichtungen geeignet, bei denen ein Flüssigkeitsvolumen mit oder ohne einer Relativgeschwindigkeit zu einem Elektronengenerator mit beschleunigten Elektronen beaufschlagt werden kann.

## Patentansprüche

1. Verfahren zum Beaufschlagen einer Flüssigkeit (11) mit beschleunigten Elektronen, **gekennzeichnet durch** folgende Schritte
a) Bereitstellen der Flüssigkeit (11),
b) Vermischen der Flüssigkeit (11) mit Partikeln (12), welche mindestens einen Leuchtstoff aufweisen, wobei zumindest einer der Leuchtstoffe ein Ion oder mehrere Ionen aus der Gruppe In⁺, Sn²⁺, Pb²⁺, Sb³⁺, Bi³⁺, Ce³⁺, Ce⁴⁺, Pr³⁺, Nd³⁺, Sm²⁺, Sm³⁺, Eu²⁺, Eu³⁺, Gd³⁺, Tb³⁺, Dy³⁺, Ho³⁺, Er³⁺, Tm²⁺, Tm³⁺, Yb²⁺, Yb³⁺, Ti³⁺, V²⁺, V³⁺, V⁴⁺, Cr³⁺, Mn²⁺, Mn³⁺, Mn⁴⁺, Fe³⁺, Fe⁴⁺, Fe⁵⁺, Co³⁺, Co⁴⁺, Ni²⁺, Cu⁺, Ru²⁺, Ru³⁺, Pd²⁺, Ag⁺, Ir³⁺, Pt²⁺ und Au⁺ beinhaltet und wobei die Partikel (12) derart ausgebildet werden, dass die Partikel (12) nach dem Vermischen dispergiert in der Flüssigkeit (11) vorliegen, wobei deren Sink- bzw. Auftriebsgeschwindigkeit innerhalb eines nicht fließenden Volumens der Flüssigkeit (11) kleiner als 100 nm/s ist, und wobei die Größe und oder die Dichte der Partikel (12) derart eingestellt wird, dass die Partikel (12) nach dem Vermischen in der Flüssigkeit (11) dispergiert vorliegen,
c) Beaufschlagen der Flüssigkeit (11) mit einer elektromagnetischen Strahlung, welche den mindestens einen Leuchtstoff zum Lumineszieren anregt, wobei Licht, welches mit einem menschlichen Auge erfassbar ist oder UV-Strahlung als elektromagnetische Strahlung verwendet wird;
d) Erfassen eines Istwertes einer die Lumineszenz des Leuchtstoffs charakterisierenden physikalischen Größe, wobei als physikalische Größe, welche die Lumineszenz des Leuchtstoffs charakterisiert, verwendet wird: die Lumineszenzlebensdauer, die Intensität mindestens einer Wellenlänge der Lumineszenz oder die Wellenlänge, bei der die Lumineszenz sein Intensitätsmaximum ausbildet,
e) Beaufschlagen der Flüssigkeit (11) mit beschleunigten Elektronen, bis der erfasste Istwert einem Sollwert entspricht.

2. Verfahren nach Anspruch 1, **dadurch gekennzeichnet, dass** die Partikel (12) aus einem Basismaterial bestehen, an dessen Oberfläche der Leuchtstoff aufgetragen und/oder in dem der Leuchtstoff eingebettet wird.

3. Verfahren nach Anspruch 2, **dadurch gekennzeichnet, dass** ein Kunststoff als Basismaterial verwendet wird.

4. Verfahren nach Anspruch 2, **dadurch gekennzeichnet, dass** ein biokompatibles Material als Basismaterial verwendet wird.

5. Verfahren nach einem der Ansprüche 2 bis 4, **dadurch gekennzeichnet, dass** der Leuchtstoff vollständig vom Basismaterial umschlossen wird.

6. Verfahren nach einem der vorhergehenden Ansprüche, **dadurch gekennzeichnet, dass** die Flüssigkeit (12) vor dem Beaufschlagen mit beschleunigten Elektronen zu einem Flüssigkeitsfilm ausgebildet wird.

## Claims

1. Method for applying accelerated electrons to a liquid (11), **characterized by** the following steps:
a) providing the liquid (11),
b) mixing the liquid (11) with particles (12) including at least one luminophore, wherein at least one of the luminophores includes one or more ions from the group of In⁺, Sn²⁺, Pb²⁺, Sb³⁺, Bi³⁺, Ce³⁺, Ce⁴⁺, Pr³⁺, Nd³⁺, Sm²⁺, Sm³⁺, Eu²⁺, Eu³⁺, Gd³⁺, Tb³⁺, Dy³⁺, Ho³⁺, Er³⁺, Tm²⁺, Tm³⁺, Yb²⁺, Yb³⁺, Ti³⁺, V²⁺, V³⁺, V⁴⁺, Cr³⁺, Mn²⁺, Mn³⁺, Mn⁴⁺, Fe³⁺, Fe⁴⁺, Fe⁵⁺, Co³⁺, Co⁴⁺, Ni²⁺, Cu⁺, Ru²⁺, Ru³⁺, Pd²⁺, Ag⁺, Ir³⁺, Pt²⁺ and Au⁺, and wherein the particles (12) are formed such that the particles (12), after the mixing, are dispersed in the liquid (11), wherein their speed of descent or ascent within a non-flowing volume of the liquid (11) is less than 100 nm/s, and wherein the size and/or density of the particles (12) is adjusted such that the particles (12) are dispersed in the liquid (11) after mixing,
c) applying electromagnetic radiation which excites the at least one luminophore to luminescence, using light detectable by a human eye or UV radiation as electromagnetic radiation to the liquid (11);
d) detecting an actual value of a physical parameter that characterizes the luminescence of the luminophore, where the physical parameter used that characterizes the luminescence of the luminophore is: the luminescence lifetime, the intensity of at least one wavelength of luminescence or the wavelength at which the luminescence forms its intensity maximum,
e) applying accelerated electrons to the liquid (11) until the actual value detected corresponds to a target value.

2. Method according to Claim 1, **characterized in that** the particles (12) consist of a base material on the surface of which the luminophore has been applied and/or in which the luminophore is embedded.

3. Method according to Claim 2, **characterized in that** a plastic is used as base material.

4. Method according to Claim 2, **characterized in that** a biocompatible material is used as base material.

5. Method according to any of Claims 2 to 4, **characterized in that** the luminophore is completely surrounded by the base material.

6. Method according to any of the preceding claims, **characterized in that** the liquid (12) is formed to a liquid film before applying accelerated electrons.

## Revendications

1. Procédé destiné à soumettre un liquide (11) à des électrons accélérés,
**caractérisé par** les étapes suivantes
a) fournir le liquide (11),
b) mélanger le liquide (11) à des particules (12) qui comportent au moins une substance luminescente, au moins une des substances luminescentes comprenant un ion ou plusieurs ions du groupe In⁺, Sn²⁺, Pb²⁺, Sb³⁺, Bi³⁺, Ce³⁺, Ce⁴⁺, Pr³⁺, Nd³⁺, Sm²⁺, Sm³⁺, Eu²⁺, Eu³⁺, Gd³⁺, Tb³⁺, Dy³⁺, Ho³⁺, Er³⁺, Tm²⁺, Tm³⁺, Yb²⁺, Yb³⁺, Ti³⁺, V²⁺, V³⁺, V⁴⁺, Cr³⁺, Mn²⁺, Mn³⁺, Mn⁴⁺, Fe³⁺, Fe⁴⁺, Fe⁵⁺, Co³⁺, Co⁴⁺, Ni²⁺, Cu⁺, Ru²⁺, Ru³⁺, Pd²⁺, Ag⁺, Ir³⁺, Pt²⁺ et Au⁺ et les particules (12) étant formées de manière à ce que les particules (12) soient présentes de manière dispersée dans le liquide (11) après mélange, leur vitesse de descente ou de soulèvement à l'intérieur d'un volume de liquide (11) sans écoulement étant inférieure à 100 nm/s, et la taille et/ou la densité des particules (12) étant réglées de manière à ce que les particules (12) soient présentes de manière dispersée dans le liquide (11) après mélange,
c) soumettre le liquide (11) à un rayonnement électromagnétique qui stimule la luminescence de l'au moins une substance luminescente, la lumière qui peut être détectée par un œil humain ou un rayonnement UV étant utilisée comme rayonnement électromagnétique ;
d) acquérir une valeur réelle d'une grandeur physique qui caractérise la luminescence de la substance luminescente, la durée de vie de la luminescence, l'intensité d'au moins une longueur d'onde de la luminescence ou la longueur d'onde à laquelle la luminescence forme son maximum d'intensité étant utilisée comme grandeur physique qui caractérise la luminescence de la substance luminescente,
e) soumettre le liquide (11) à des électrons accélérés jusqu'à ce que la valeur réelle acquise corresponde à une valeur de consigne.

2. Procédé selon la revendication 1, **caractérisé en ce que** les particules (12) sont en une matière de base, sur la surface de laquelle la substance luminescente est appliquée et/ou dans laquelle la substance luminescente est incorporée.

3. Procédé selon la revendication 2, **caractérisé en ce qu'**une matière synthétique est utilisée comme matière de base.

4. Procédé selon la revendication 2, **caractérisé en ce qu'**une matière biocompatible est utilisée comme matière de base.

5. Procédé selon l'une des revendications 2 à 4, **caractérisé en ce que** la substance luminescente est entièrement entourée par la matière de base.

6. Procédé selon l'une des revendications précédentes, **caractérisé en ce que** le liquide (12) est mis sous la forme d'un film liquide avant d'être exposé à des électrons accélérés.
